# EUROPEAN PATENT SPECIFICATION

(11) **EP 2 938 325 B1**
(45) Date of publication and mention of the grant of the patent: **10.04.2019**
(21) Application number: 13830158.5
(22) Date of filing: 27.12.2013
(51) Int. Cl.: A61K 9/08, A61K 47/10, A61K 31/196, A61P 29/00

(54) **DICLOFENAC COMPOSITION**
DICLOFENACZUSAMMENSETZUNG
COMPOSITION DE DICLOFÉNAC

(30) Priority: 28.12.2012 IN 3704MU2012
(43) Date of publication of application: 04.11.2015
(73) Proprietor: Themis Medicare Limited, Goregaon (W), Mumbai 400 104 (IN)
(72) Inventor: PATEL, Dinesh Shantilal, Mumbai 400 104 Maharashtra (IN); PATEL, Shachin Dinesh, Mumbai 400 104 Maharashtra (IN); KURANI, Shashikant Prabhudas, Mumbai 400 104 Maharashtra (IN); PATEL, Madhavlal Govindlal, Mumbai 400 104 Maharashtra (IN)
(74) Representative: HGF Limited
(86) International application number: PCT/IN2013/000805
(87) International publication number: WO 2014/102824

(56) References cited:
- WO-A2-2005/027977
- WO-A2-2006/126214
- US-A1- 2008 153 914
- M. MANCONI ET AL: "Penetration enhancer-containing vesicles: Composition dependence of structural features and skin penetration ability", EUROPEAN JOURNAL OF PHARMACEUTICS AND BIOPHARMACEUTICS, vol. 82, no. 2, 1 October 2012 (2012-10-01), pages 352-359, XP055115170, ISSN: 0939-6411, DOI: 10.1016/j.ejpb.2012.06.015
- "Final Report - Measurement of Pain Sensitivity (Threshold) at Aquadol Injection Site in Wistar Rats by Intramuscular Route", 25 June 2014 (2014-06-25), Spira Labs Limited
- NICOLL LESLIE H ET AL: "Intramuscular injection: an integrative research review and guideline for evidence-based practice.", APPLIED NURSING RESEARCH : ANR AUG 2002, vol. 15, no. 3, August 2002 (2002-08), pages 149-162, ISSN: 0897-1897
- "Propylene Glycol" In: "Handbook of Pharmaceutical Excipients", 2009 pages 592-594,
- "Polyethylene Glycol" In: "Handbook of Pharmaceutical Excipients", 2009 pages 517-522,
- Aparacio RM et al.: "In vitro studies of the hemolytic activity of microemulsions in human erythrocytes", ScienceDirect Journal of Pharmaceutical and Biomedical Analysis, vol. 39, no. 5 28 July 2005 (2005-07-28), Retrieved from the Internet: URL:http://www.sciencedirect.com/science/a rticle/pii/S0731708505004139 [retrieved on 2016-06-17]
- Mottu F et al.: "Comparative Hemolytic Activity of Undiluted Organic Water-Miscible Solvents for Intravenous and Intra-Arterial Injection", journal.pda.org PDA Journal of Pharmaceutical Science and Technology, vol. 55, no. 1 2001, Retrieved from the Internet: URL:http://journal.pda.org/content/55/1/16 .abstract?ijkey=e8b62278383a2566ddc69d24ac 9947b6f94e92bb&keytype2=tf_ipsecsha [retrieved on 2016-06-20]
- "Glycofurol" In: "Handbook of Pharmaceutical Excipients", 2009 pages 297-298,

## Description

### Field of invention

The present invention relates to a composition comprising Diclofenac and salts thereof. In particular, the invention provides a composition comprising 25 - 200 mg/mL of Diclofenac or salts thereof for the parenteral administration through intramuscular, intravenous route. The invention also provides compositions comprising a combination of Diclofenac and other drugs. The invention further provides a method for preparing said composition.

### Background of Invention

Diclofenac possesses structural characteristics of the arylalkanoic acid agents and displays anti-inflammatory, analgesic, and anti-pyretic activity. Diclofenac is unique among the non-steroidal anti-inflammatory drugs (NSAIDs) in that it possesses the mechanism of action for the inhibition of arachidonic acid cyclooxygenase system, lipooxygenase pathway, arachidonic acid release resulting in decreased production of prostaglandins and thromboxanes, leukotrienes and reduction of arachidonic acid, respectively (Martindale, 2002).

Both salts of Diclofenac i.e., Diclofenac Sodium and Diclofenac Potassium and alike salts share almost the same physicochemical properties except their molecular weights. It can be also employed and used as diethylamine salt or diethanolamine or beta-dimethyl aminoethanol salt.

Diclofenac is available in 120 different countries and is perhaps the most widely used NSAIDs in the world, being the 8^{th} largest selling drug overall.

It is used mainly (as the sodium salt and potassium salt) for the relief of pain and inflammation in various conditions: musculoskeletal and joint disorders such as rheumatoid arthritis, osteoarthritis and ankylosing spondylitis, peri-articular disorders such as bursitis and tendinitis, soft-tissue disorders such as sprains and strains, and other painful conditions such as renal colic, acute gout, dysmenorrhoea, migraine, and after some surgical procedures. It has also been used in some countries for the management of actinic keratosis and fever. Eye drops of Diclofenac Sodium are used for the prevention of intra-operative miosis during cataract extraction, for the treatment of inflammation after surgery or accidental trauma, and for the relief of ocular signs and symptoms of seasonal allergic conjunctivitis.

The usual oral or rectal dose of Diclofenac Sodium is 75 to 150 mg daily in divided doses. In the U.K., the maximum dose regardless of route or indications is 150 mg daily; however, in the U.S.A, a maximum oral dose of 200 mg daily is allowed in the treatment of rheumatoid arthritis. Modified-release preparations of Diclofenac Sodium are available for oral use. Diclofenac has also been given in equivalent oral doses as the free acid in tablet forms as dispersible preparations for short-term treatment up to 3 months period. Diclofenac is also given orally as the potassium salt. Doses of the potassium salts are also used in the treatment of migraine in an initial dose of 50 mg taken at the first signs of an attack, an additional dose of 50 mg may be taken after 2 hours if symptoms persist. If necessary further doses of 50 mg may be taken after 2 hours if symptoms persist. If necessary further doses of 50 mg may be taken every 4 to 6 hours to a maximum daily dose of 200 mg.

Diclofenac Sodium may also be given by deep intramuscular injection into the gluteal muscle in a dose of 75 mg once daily or, if required in severe conditions, 75 mg twice daily. Diclofenac Sodium may also be given as a continuous or intermittent intravenous infusion in glucose. 5% or sodium chloride 0.9% (both previously buffered with sodium bicarbonate) or as a bolus intravenous injection. For the treatment of postoperative paid a dose of 75 mg may be given over 30 to 120 minutes or as a bolus injection. The dose may be repeated once after 4 to 6 hours if necessary. To prevent postoperative pain, as initial dose of 25 to 50 mg Diclofenac Sodium may be given after surgery over 15 to 60 minutes followed by 5 mg / hour to a maximum of 150 mg daily. Alternatively, the initial dose may be given as a bolus injection over 5 to 60 seconds followed by additional injections up to the maximum daily dosage; this may be repeated after 4 to 6 hours if necessary although the total dose should not exceed the maximum daily dose of 150 mg. The maximum period recommended for parenteral use is 2 days. Diclofenac Sodium is also used intramuscularly in renal colic in a dose of 75 mg repeated once after 30 minutes if necessary (Martindale, 36^{th} edition).

Conventionally formulated Diclofenac Sodium injections are limited to intramuscular administration. This limitation has arisen, not as a consequence of the intravenous safety profile, but principally due to the physico-chemical properties of the drug. Poor aqueous solubility of the Sodium salt of Diclofenac has a particularly high tendency to crystallize from aqueous and organic solutions. Physically stable solutions containing at least 25 mg/ml of Diclofenac Sodium necessitates the use of potent solubilizing co solvents, such as Macrogols and Benzyl alcohol. These co-solvents have an unfavorable intravenous safety profile and are associated with venous sequelae, high hemolytic and sensitizing (Reed, K.W. et al, J. Par. Sci: Technol. 39 (2) (1985) 64-68).

Further the high pH of the marketed Diclofenac product requires rendering Diclofenac Sodium soluble and the hyper-osmolar nature of the formulation contribute to the discomfort which is frequently experienced at the site of the injection when administered intramuscularly.

Further, use of the excipients like Propylene glycol is also known to cause pain at the site of injection.

There have been many efforts for the modification in formulation of Diclofenac. There are number of documents that mention modification in the composition of Diclofenac. Due to limited solubility of Diclofenac Sodium in water, numbers of attempts are being made to solubilize the same and then administer for treatment of mammals for various pathological conditions

US 4,614,741 teaches about the depot injectable containing anti-inflammatory agents like Diclofenac or Diclofenac Sodium, which was prepared in 10-60% of suspending agents selected from the group consisting of biscoleo, isopropyl myristate, ethyl oleate, castor oil, sesame oil, arachis oil, cottonseed oil, almond oil, olive oil, neatsfoot oil, neutral oil and maize oil, for intramuscular administration. However, use of such suspending agent provides painful and viscous injection that causes swelling or pain at the site of injection.

US 4,711,906 discloses an aqueous, stable, relatively concentrated solution of Diclofenac, which contain a mixture of propylene glycol and polyethylene glycol in defined quantitative proportions. The solutions preferably contain a local anesthetic such as lidocaine and a reducing agent as stabilizer. However, use of propylene glycol makes the injection painful and lignocaine is added to alleviate such painful administration.

US 2005/0238674 A1 describes a stable injectable compositions comprising: either (a) Diclofenac or a pharmaceutically acceptable Diclofenac salt and a cyclodextrin, or (b) an inclusion complex of Diclofenac or a pharmaceutically acceptable Diclofenac salt and a cyclodextrin, or a mixture of (a) and (b), for intramuscular and intravenous administration. The solutions contain Diclofenac or Diclofenac salt, cyclodextrin, and an antioxidant selected from Monothioglycerol, or a combination of ethylene diamine tetra-acetic acid and N-acetyl-cysteine. It is observed that elimination of cyclodextrin is a problem in renal compromised patient.

US 5,389,681 discloses a pharmaceutical composition in the form or a sterilizable parenteral solution comprising a Diclofenac salt and stabilizers, such as ethyl lactate combined with glutathione or N-acetylcysteine. Glutathione are selected derivative of amino acids are being very costly thereby increases the basic cost of the injection.

US 5,283,067 discloses a lyophilized dry formulation suitable for the preparation of a stable, aqueous suspension for the parenteral administration of a Diclofenac salt. The dry formulation contains a pharmaceutically acceptable and micronized salt of Diclofenac and optional pharmaceutically acceptable adjuvants.

US 2011/0275717 A1 discloses a pharmaceutical formulation comprising a pharmaceutically acceptable salt of Diclofenac, at least one polyoxyalkylene ester of a hydroxyl fatty acid, water, and, optionally, a co-solvent. This composition has a limitation with use of fatty acid derivative and requires special carriers.

US 5679660 A teaches about the method of preparing an injectable pharmaceutical or veterinary composition which comprises either Diclofenac or a salt thereof and 2-hydroxypropyl beta-cyclodextrin, or an inclusion complex of Diclofenac or a salt thereof and 2-hydroxypropyl beta-cyclodextrin with preferred concentration of Diclofenac of 25 mg/ml. The volume of injection prepared by this method contains 75mg/3ml Diclofenac which can be painful as IM dosage form. The use of beta cyclodextrin has also toxicity problem which is always questioned for IV administration.

US20080153914 discloses injectable formulations of water-soluble salts of diclofenac in Glycofurol. However, Glycofurol is known as a tissue irritant.

WO 2006/126214 discloses injectable compositions comprising diclofenac and a solvent system comprising a mixture of glycols.

It is evident from the details mentioned above that Diclofenac injection formulation and its manufacturing is still a challenging task. There is a continuous need of a composition which is medically useful, beneficial and economic. The same has motivated the inventors to find out the simple process to find the manufacturing process which is simple and economic, using the cheaper excipients and still beneficial to patients physiologically without causing any discomfort.

It is an object of the invention to provide an economical yet physiologically effective composition comprising Diclofenac or salts thereof.

Another object of the invention is to provide a composition comprising Diclofenac suitable to be given through multiple routes viz., intramuscular or intravenous routes of administration.

Another object of the invention is to provide a composition comprising Diclofenac or salts thereof in combination of other anti-inflammatory, analgesic, and/ or anti-pyretic agent.

Yet another object of the invention is to provide a patient compliant aqueous composition of Diclofenac which is not irritating or painful and does not cause hemolysis.

Still another object of the invention to provide a composition comprising Diclofenac with less toxicity and more bioavailability

### Summary of Invention

The present invention provides an aqueous injectable composition comprising 25-200mg/mL of Diclofenac or salts thereof, 5-50% (wt/v) of diethylene glycol monoethyl ether and water as a principal solvent in at least 0.5ml of solution, wherein said composition has a viscosity between about 1 to about 5 mPa·s (cps) at 30°C. The invention also provides such a composition comprising Diclofenac in combination with other pharmaceutically active ingredients selected from anti-inflammatory, analgesic, and/ or anti-pyretic agents.

In an embodiment, the composition comprises Diclofenac or salts thereof in an amount of 25 - 200 mg. Diclofenac salt is selected from Diclofenac sodium, Diclofenac potassium, Diclofenac diethylamine, Diclofenac diethanolamine or Diclofenac beta-dimethyl aminoethanol.

In another embodiment, the composition comprises solvents/ co-solvents and preservatives. The solvents/ co-solvents and preservatives may be selected from, but not limited to, alcohols, parabens, sodium metabisulphite, sodium bisulphate, sodium sulphite, propylene glycol, thiomerosal, glycerol and thioglycerol or a combination thereof, ascorbyl palmitate, ascorbate, tocopherol alpha, alpha-tocopherol hydrogen succinate and mixture of tocopheryl derivatives, thioglycolate sodium.

In another embodiment, the amount of preservative sodium metabisulphite in the composition is between 0.1 - 1 %.

In another embodiment, the composition comprises benzyl alcohol not more than 3%.

In another embodiment, the composition also comprises chelating agents. The chelating agent may be selected from, but not limited to, sodium EDTA (ethylenediaminetetra acetic acid), disodium EDTA, Calcium disodium EDTA, Calcium versetamide Na, Calteridol, DTPA (Diethylenetriaminepenta acetic acid). The amount of chelating agent used is between 0.01-0.05 %.

In another embodiment, the composition has a pH value between 7.5 - 9.0. The composition may be diluted and may be filled as 1-20 ml solution volume in ampoules or multi dose vials. In preferred embodiment, the composition is prepared in 1-5 ml of solution and more preferably in 1-3 ml of solution.

In yet another embodiment, the invention provides an injectable aqueous composition comprising 25-200 mg of Diclofenac Sodium in 5- 50 % of Transcutol in combination with water as a principal solvent in 1ml of solution.

In another embodiment, the composition may be added to infusion bags/bottles by suitably diluting with infusion liquid.

In another aspect, the invention provides a method for preparation of an aqueous composition as defined above. The method comprises preparing an aqueous solution of Transcutol by adding 30-50 % of water to 5-50% of Transcutol; adding 25 mg-200mg/mL Diclofenac in said aqueous solution with constant stirring under nitrogen atmosphere; adjusting pH of said solution in a range of 7.5-9.0; and diluting said solution to get desired concentration of Diclofenac.

In another aspect, the invention provides the above composition for use in the treatment or prevention of physiological disorders/disease that may be benefited with the composition of Diclofenac Sodium such as various musculoskeletal and joint disorders like rheumatoid arthritis, osteoarthritis, ankylosing spondylitis, peri-articular disorders like bursitis and tendinitis, soft tissue disorders like sprains and strains, painful condition such as renal colic, acute gout, dysmenorrhoea, migraine and some surgical procedures, in management of actinic keratosis and fever, post operative pain, for juvenile idiopathic arthritis, acute postoperative pain, intra-operative miosis, for treatment of inflammation after surgery.

In still another aspect, the invention provides the above composition for use in the treatment or prevention of headache, sore throat, various musculoskeletal and joint disorders like rheumatoid arthritis, osteoarthritis, ankylosing spondylitis, peri-articular disorders like bursitis and tendinitis, soft tissue disorders like sprains and strains, painful condition such as renal colic, acute gout, dysmenorrhoea, migraine and some surgical procedures, in management of actinic keratosis and fever, post operative pain, for juvenile idiopathic arthritis, acute postoperative pain, intra-operative miosis, for treatment of inflammation after surgery, pain control of total hip replacement arthroplasty.

### Detailed Description of the Invention

The invention provides a composition comprising Diclofenac or salt thereof as defined in claim 1. Diclofenac salt is selected from Diclofenac sodium, Diclofenac potassium, Diclofenac diethylamine, Diclofenac diethanolamine or Diclofenac beta-dimethyl aminoethanol. Conventionally formulated Diclofenac Sodium injections are limited to intramuscular administration. This limitation has arisen, not as a consequence of the intravenous safety profile, but principally due to the physico-chemical properties of the drug. Poor aqueous solubility of the Sodium salt of Diclofenac has a particularly high tendency to crystallize from aqueous and organic solutions. The invention provides therapeutically effective amount of Diclofenac or salts thereof in an aqueous composition suitable to be administered by multiple routes.

In particular, the invention provides a composition comprising Diclofenac Sodium in a concentration of 25-200 mg/mL for parenteral through IM/IV route. The composition preferably comprises Diclofenac Sodium and Transcutol. The amount of Diclofenac Sodium varies between 25-200 mg in 0.5 to 20 ml solutions, preferably 1-5 ml solutions, and more preferably 1-3 ml solutions.

The concentration of Transcutol used is between 5-50 % (w/v). Transcutol is diethylene glycol monoethyl ether. Transcutol is not known to cause any toxicity related issues including irritancy or pain for a therapeutic applications. Whereas other excipients used for preparing aqueous Diclofenac Sodium compositions like Glycofurol is known as a tissue irritant and Cremophor EL is known to cause anaphylactic shocks due to its tendency to cause histamine production when injected. The safety profile of Transcutol in humans for its use has been exhaustively studied. Its' LD₅₀ in rat through IV route is 4 g/kg, intraperitoneal: mouse LD₅₀ is 2300 mg/kg, subcutaneous : mouse 5500 µl/kg, intravenous :mouse 4300 µl/kg, LD₅₀; intravenous : Dog 3 ml/kg LD₅₀; intravenous : rabbit 2500 µl/kg, LD₅₀; eyes: rabbit 500 mg. Transcutol occurs as colorless and transparent *liquid* well miscible with water. In the present invention, it is preferable to use diethylene glycol monoethyl ether having a purity of 99% or higher, more preferably 99.7% of higher and most preferably 99.9% or higher.

The density of Transcutol is 0.988. Transcutol makes the composition of the present invention less viscous. It enhances and offers an advantage of better absorption of drug in mammals when injected and hence has better pharmacological effect for the intended purpose. The composition prepared in accordance with the present invention has a viscosity from about 1- about 5 mPa·s (cps) at 30°C. The prepared Diclofenac injection composition is easy to be injected and offers less pressure when administered intra muscularly. It can be administered with ease while administering in to the tissues by the health workers, thereby causing less pain and less pressure. Also, while testing it was observed that Transcutol offers less toxicity at the administered dose and is safe without any cause or concern anatomically or physiologically.

In some embodiments, the invention provides compositions comprising additional excipients e.g. preservatives. In another embodiment, the composition comprises benzyl alcohol at a concentration in the range of 1-15 %. In preferred embodiment, benzyl alcohol is used between 1-3%. The other preservatives used, but not limited to, are sodium metabisulphite [0.1-1%], ethanol, chlorobutanol [0.5%], parabens like methylparaben [0.1-0.18%], propylparaben [0.01-0.02%], sodium bisulphite [0.02-0.66%], sodium sulphite [0.05-0.2%], propylene glycol, thiomerosal < or = [0.01%], glycerol and/or thioglycerol, phenol [0.5%], chlorocresol [0.1-0.3%], methyl hydroxybenzoate [0.1-0.2% w/v], ascorbyl palmitate, ascorbate [0.1-4.8% w/v], butylated hydroxyanisole (BHA) and butylated hydroxytoluene (BHT) can also be added.

In certain embodiments, the composition comprises antioxidants selected from but not limited to thioglycerols, acetyl cysteine, butylated hydroxyanisole (BHA) and butylated hydroxytoluene (BHT), ascorbates, ascorbyl palmitate, methylparaben, Propylparaben, thiomersal and mixed Tocopheryl ingredient.

The pH is critical for maintaining stability of the Diclofenac injection hence pH is maintained at 7.5 to 9 using suitable buffering agents and alkalizer. The buffering agents may be selected from, but not limited to alkali metal hydroxides like sodium hydroxide, potassium hydroxide, tri sodium citrate, sodium phosphate salts like monosodium phosphate salt or disodium phosphate salt, potassium phosphate salts like mono or di potassium phosphate salt, sodium acetate, Tris buffers or other alkaliser are used for adjusting pH in the desired range.

The chelating agents may be added optionally to chelate the traces of metallic impurity and to maintain the stability for longer duration. The chelating agent used, but not limited to, are sodium EDTA (ethylene diamine tetra acetic acid) [0.01-0.2%], disodium EDTA [0.01-0.11%], Calcium disodium EDTA [0.01-0.1%], Calcium versetamide Na [2.84%], Calteridol [0.023%], DTPA (diethylenetriaminepenta acetic acid) [0.04-1.2%] can be used.

Water is added in the composition in quantity sufficient (q.s.) to make it 0.5 ml or 1 ml or to give different volumes for different strength. The injectable composition may be filled in ampoule and vials after aseptic filtration and flushed under nitrogen blanket. The composition may be standardized by aseptic filtration or suitable methods like gamma irradiation or terminal sterilized. The injections so formed are stable and may diluted further in infusion liquid to obtain the desired strength of drug or without diluting further by intramuscular and as slow bolus intravenous or suitably adding to infusion liquid as per physician need.

The composition may also comprise Diclofenac in combination with other pharmaceutically active ingredients such as other anti-inflammatory, analgesic, and/ or anti-pyretic agents.

The present invention provides an aqueous formulation and may be given as infusion after diluting with saline, glucose, dextrose or Ringer's solution. The composition may be diluted with water to use directly or by adding to the infusion or can be injected through IM/IV route. It may be given to humans in deltoid muscles.

In another aspect, the invention provides a method for preparing compositions comprising Diclofenac or salts thereof. Sodium. In a suitable vessel, double distilled water is previously heated at 80 to 85°C and is brought to the room temperature. In about 50% of the composition mixture, water in quantity ranging from 30 to 50 % is mixed with the 5-50 % of Transcutol and 1-15% of benzyl alcohol. To this mixture, Diclofenac sodium is added with constant stirring, under the nitrogen bubbling, followed by addition of suitable buffering agents like Tris buffer or alkalizer sodium hydroxide to adjust the pH of solution in the range of 7.5 to 9.0. The solution is further diluted with sufficient quantity of double distilled water to the required volume for the desired final concentration of Diclofenac sodium. The process is carried under the constant nitrogen flushing to replace the level to minimum amount of the oxygen. The final composition is filtered aseptically using 0.22 micron filter or terminally sterilized to free the composition from endotoxins. The injections are stable at accelerated conditions for more than 6 months at 40°C/75 % RH kept at ICH stability conditions.

The solution is maintained under the nitrogen blanket and is stable under storage viscosity of the solution is found to be desirable for use by workers for user friendly injectables. This solution can be used for I.M or I.V use. The advantage of these injections is that it causes less pain as compared to marketed formulations. Similarly different strength of solution of Diclofenac Sodium i.e. 25 mg, 37.5mg, 50 mg, 100 mg, 150 mg, 175 mg and 200mg are prepared per 0.5, 1ml, 2ml, 3 ml, 4ml 5 ml or their multiple doses as per given process and may be used as injectable liquid for administration through I.M or I.V route in the mammals.

Some of the formulations prepared in accordance with the present invention are given below in Tables 1-2 and examples-:

### Tables 1a-1g: Formulations prepared using Diclofenac Sodium in various concentrations

**Table 1a**

| **Diclofenac sodium:** 25 to 200 mg/ml (2.5 to 20%) | | | | | |
|---|---|---|---|---|---|
| **Transcutol :** 5 to 50%, **Benzyl alcohol** : 1 to 15%, **Water for injection** : q.s (quantity sufficient) | | | | | |
| **Different strength of Diclofenac sodium** | **Diclofenac Sodium strength** | **No of experiments** | **Transcutol (%) range** | **benzyl alcohol (%) range** *(0%* = *without benzyl alcohol)* | **Water for Injection range** |
| A | **25mg** | 1 | 5% | 0% | q.s |
| | | 2 | 5% | 2% | q.s |
| | | 3 | 20% | 0% | q.s |
| | | 4 | 20% | 2% | q.s |
| | | 5 | 20% | 15% | q.s |
| | | 6 | 30% | 0% | q.s |
| | | 7 | 30% | 2% | q.s |
| | | 8 | 30% | 15% | q.s |

**Table 1b**

| **Different strength of Diclofenac sodium** | **Diclofenac Sodium strength** | **No of experiments** | **Transcutol (%) range** | **benzyl alcohol (%) range** (0% = without benzyl alcohol) | **Water for Injection range** |
|---|---|---|---|---|---|
| B | **50 mg** | 1 | 5% | 2% | q.s |
| | | 2 | 5% | 15% | q.s |
| | | 3 | 20% | 0% | q.s |
| | | 4 | 20% | 2% | q.s |
| | | 5 | 20% | 15% | q.s |
| | | 6 | 30% | 2% | q.s |
| | | 7 | 30% | 15% | q.s |
| | | 8 | 50% | 15% | q.s |

**Table 1c**

| **Different strength of Diclofenac sodium** | **Diclofenac Sodium strength** | **No of experiments** | **Transcutol (%) range** | **benzyl alcohol (%) range** *(0%* = *without benzyl alcohol)* | **Water for Injection range** |
|---|---|---|---|---|---|
| C | **75 mg** | 1 | 20% | 15% (with 0.02% sodium metabisulphite) | q.s |
| | | 2 | 20% | 15% (without sodium metabisulphite) | q.s |
| | | 3 | 50% | 0% | q.s |
| | | 4 | 50% | 15% (with 0.02% sodium metabisulphite) | q.s |
| | | 5 | 50% | 15% (without sodium metabisulphite) | q.s |

**Table 1d**

| **Different strength of Diclofenac sodium** | **Diclofenac Sodium strength** | **No of experiments** | **Transcutol (%) range** | **benzyl alcohol (%) range** *(0%* = *without benzyl alcohol)* | **Water for Injection range** |
|---|---|---|---|---|---|
| D | **100 mg** | 1 | 5% | 10% | q.s |
| | | 2 | 5% | 15% (with 0.02% sodium metabisulphite) | q.s |
| | | 3 | 10% | 15% (with 0.02% sodium metabisulphite) | q.s |
| | | 4 | 15% | 10% | q.s |
| | | 5 | 20% | 2 % | q.s |
| | | 6 | 20% | 10% | q.s |
| | | 7 | 20% | 15% | q.s |
| | | 8 | 30% | 0% | q.s |
| | | 9 | 30% | 2% | q.s |
| | | 10 | 30% | 10% | q.s |
| | | 11 | 30% | 15% | q.s |
| | | 12 | 50% | 0% | q.s |
| | | 13 | 50% | 2% | q.s |
| | | 14 | 50% | 10% | q.s |
| | | 15 | 50% | 15% | q.s |

**Table 1e**

| **Different strength of Diclofenac sodium** | **Diclofenac Sodium strength** | **No of experiments** | **Transcutol (%) range** | **benzyl alcohol (%) range** *(0%* = *without benzyl alcohol)* | **Water for Injection range** |
|---|---|---|---|---|---|
| E | **150 mg** | 1 | 5% | 15% | q.s |
| | | 2 | 20% | 4% | q.s |
| | | 3 | 20% | 8% | q.s |
| | | 4 | 20% | 15% | q.s |
| | | 5 | 30% | 0% | q.s |
| | | 6 | 30% | 2% | q.s |
| | | 7 | 30% | 4% | q.s |
| | | 8 | 30% | 8% | q.s |
| | | 9 | 30% | 15% | q.s |
| | | 10 | 50% | 0% | q.s |
| | | 8 | 30% | 0% | q.s |
| | | 9 | 30% | 2% | q.s |
| | | 10 | 30% | 10% | q.s |
| | | 11 | 30% | 15% | q.s |
| | | 12 | 50% | 0% | q.s |
| | | 13 | 50% | 2% | q.s |
| | | 14 | 50% | 10% | q.s |
| | | 15 | 50% | 15% | q.s |

**Table 1f**

| **Different strength of Diclofenac sodium** | **Diclofenac Sodium strength** | **No of experiments** | **Transcutol (%) range** | **benzyl alcohol (%) range *(0%* = *without benzyl alcohol)*** | **Water for Injection range** |
|---|---|---|---|---|---|
| F | **175mg** | 1 | 5% | 15% | q.s |
| | | 2 | 20% | 4% | q.s |
| | | 3 | 20% | 8% | q.s |
| | | 3 | 20% | 15% | q.s |
| | | 4 | 30% | 0% | q.s |
| | | 5 | 30% | 2% | q.s |
| | | 6 | 30% | 4% | q.s |
| | | 7 | 30% | 8% | q.s |
| | | 8 | 30% | 15% | q.s |
| | | 9 | 50% | 0% | q.s |
| | | 10 | 50% | 2% | q.s |
| | | 11 | 50% | 4% | q.s |
| | | 12 | 50% | 8% | q.s |
| | | 13 | 50% | 15% | q.s |

**Table 1g**

| **Different strength of Diclofenac sodium** | **Diclofenac Sodium strength** | **No of experiments** | **Transcutol (%) range** | **benzyl alcohol (%) range *(0% = without benzyl alcohol)*** | **Water for Injection range** |
|---|---|---|---|---|---|
| G | **200 mg** | 1 | 5% | 15% | q.s |
| | | 2 | 20% | 2% | q.s |
| | | 3 | 20% | 15% | q.s |
| | | 4 | 30% | 2% | q.s |
| | | 5 | 30% | 2% | q.s |
| | | 6 | 30% | 8% | q.s |
| | | 7 | 30% | 15% | q.s |
| | | 8 | 50% | 0% | q.s |
| | | 9 | 50% | 2% | q.s |
| | | 10 | 50% | 4% | q.s |
| | | 11 | 50% | 8% | q.s |
| | | 12 | 50% | 15% | q.s |

**Table 2a : Formulations using Diclofenac Sodium in concentration 7.5%**

| **Formulation no.** | **Diclofenac Sodium concentration (%)** | **Transcutol concentration (%)** | **Benzyl alcohol concentration (%)** | **Water for injection** |
|---|---|---|---|---|
| 1 | 7.5 | 50 | 2 | q.s |
| 2 | 7.5 | 30 | 2 | q.s |
| 3 | 7.5 | 30 | 0 | q.s |
| 4 | 7.5 | 25 | 4 | q.s |
| 5 | 7.5 | 25 | 0 | q.s |
| 6 | 7.5 | 20 | 4 | q.s |
| 7 | 7.5 | 20 | 2 | q.s |
| 8 | 7.5 | 20 | 0 | q.s |
| 9 | 7.5 | 15 | 4 | q.s |
| 10 | 7.5 | 15 | 2 | q.s |
| 11 | 7.5 | 10 | 10 | q.s |
| 12 | 7.5 | 10 | 2 | q.s |
| 13 | 7.5 | 5 | 4 | q.s |
| 14 | 7.5 | 20 | 15 | q.s |
| 15 | 7.5 | 50 | 0 | q.s |

Solutions of different concentration of Diclofenac Sodium given in the tables 1-2 can be filled in volume of 0.5 ml, 1ml, 2ml, 5ml, 10 ml and 20 ml ampoules and can also be filled into multi dose vials for the therapeutic purpose required by the physicians.

Similarly different strength of solution of Diclofenac Sodium i.e. 25 mg/ml, 50 mg/ml, 100 mg/ml, 150 mg/ml, 175 mg/ml and 200mg/ml were prepared as per given process. These solutions may be used as injectable liquid for administration through I.M or I.V route in the mammals by filling in different volumes.

**Table 2 b: Formulations using Diclofenac Sodium in concentrations 2.5%, 5%, 10%, 15%, 17.5% and 20%:**

| **Formulation No.** | **Diclofenac Sodium concentration (%)** | **Transcutol concentration (%)** | **Benzyl alcohol concentration (%)** | **Water for injection** |
|---|---|---|---|---|
| 16 | 2.5 | 30 | 0 | q.s |
| 17 | 2.5 | 20 | 2 | q.s |
| 18 | 2.5 | 5 | 15 | q.s |
| 19 | 5.0 | 50 | 15 | q.s |
| 20 | 5.0 | 20 | 2 | q.s |
| 21 | 10.0 | 30 | 0 | q.s |
| 22 | 10.0 | 20 | 2 | q.s |
| 23 | 10.0 | 5 | 10 | q.s |
| 24 | 15.0 | 50 | 0 | q.s |
| 25 | 15.0 | 20 | 4 | q.s |
| 26 | 15.0 | 5 | 15 | q.s |
| 27 | 17.5 | 50 | 0 | q.s |
| 28 | 17.5 | 30 | 15 | q.s |
| 29 | 17.5 | 20 | 8 | q.s |
| 30 | 20.0 | 20 | 2 | q.s |
| 31 | 20.0 | 20 | 15 | q.s |
| 32 | 20.0 | 50 | 0 | q.s |

Similarly further formulations are prepared by additional excipients like antioxidant Sodium metabisulphite in variable concentration in addition to presence of Benzyl alcohol, alkalizer or buffers and chelating agent (table 3).

In another aspect, the invention provides a composition for use in treating certain diseases and disorders. The composition of present invention may be administered in patients suffering from pain related to osteoarthritis, joint pains, colic pains, renal colic pains induced due to stone, joint pains due to sprain or injury and also for relieving pain due to surgery, due to volunteer pain or non-volunteer pain, pain in cancer patients and to ameliorate or prevent pathological conditions. The composition may be given in a physiological disorder that can be ameliorated treated/prevented using Diclofenac.

The present invention is described with reference to the following examples, which are given by way of illustration and should not be construed to limit the scope of the present invention. The solutions compounded in the following illustration for Diclofenac sodium or as combination of Diclofenac Sodium and Paracetamol or Diclofenac Sodium and Diclyclomine HCl may be prepared for injectables.

### Example 1

In a suitable vessel, the solution is prepared comprising of Diclofenac Sodium at a concentration of 7.5% by addition of 50 % Transcutol and 2% Benzyl alcohol into previously boiled and cooled water and stirred well till the API get well dissolved. The pH of solution is adjusted in required range if required, by suitable buffering agent like Tris buffer, phosphate buffers like sodium phosphate and optionally other alkalizer like sodium hydroxide which can be used. The solution is diluted further till final volume by sufficient quantity of water. The final volume solution is sterilized aseptically and poured into ampoules & multi dose vials under continuous nitrogen blanket.

This prepared solution consists about 52% of nonaqueous solution and 48 % of aqueous solution. Similarly solution with 20% Diclofenac Sodium is prepared which is equivalent to 200 mg/ml solution.

### Example 2

At 80- 85°C, water is heated in a suitable vessel and cooled to the room temperature. The solution is prepared by dissolving 7.5% Diclofenac Sodium with 30 % Transcutol and 2% Benzyl alcohol into 50% water and stirred well continuously till the clear solution is obtained. The pH is adjusted in desired range 7.5 to 9 by suitable buffering agent like Tris buffer, phosphate buffers like sodium phosphate or optionally by other alkalizer. The solution is diluted further q.s. water to the volume. The final volume of solution is sterilized through aseptic filtration using 0.22 micron filter and poured into ampoules & multi dose vials. The process is performed under constant nitrogen flush.

This prepared solution consists about 32 % of nonaqueous solution and 68 % of aqueous solution.The resultant liquid containing 75 mg of Diclofenac Sodium in 1ml can be administered through I.M and I.V route by injection.

### Example 3

In a suitable vessel, 30 % of Transcutol is added into 50 % of water which was allowed to cool at room temperature which was initially heated at 80 to 85 °C. The solution is then stirred well with the addition of Diclofenac Sodium at a concentration of 7.5 %. After mixing the ingredient in the solution, it is further diluted with water in sufficient quantity upto required final volume such that the final liquid consists of 75 mg Diclofenac Sodium in 1ml. This resultant liquid is filtered aseptically by using 0.2 micron membrane. The pH of the solution is maintained into the range of 7.5 to 9 required by addition of suitable alkalizer or buffering agents like Tris buffer or phosphate buffers like sodium phosphate, potassium phosphate. The entire process is carried out in nitrogen blanket to remove the oxygen.

This prepared solution consists about 30 % of nonaqueous solution and 70 % of aqueous solution. The resultant solution is ready to be filled into ampoules and multidose vials. The viscosity of the solution is also found about 3.01 mPa·s (cps) at 30°C.

This can be used as an injection for administration through I.M or I.V. Similarly 15% Diclofenac Sodium containing solution is prepared as above which contains 150 mg/ml of Diclofenac Sodium.

### Example 4

Water heated at 80- 85°C, is cooled at room temperature in a suitable vessel. 4 % of Benzyl alcohol and 25 % of Transcutol is added into the above 50 % of water. Solution is stirred by adding Diclofenac Sodium at a concentration of 7.5 % till it gets dissolved. The pH of the solution is maintained at the range of 7.5 to 9 by using any suitable buffers like Tris buffer or phosphate buffers like sodium phosphate like monosodium phosphate/ disodium phosphate optionally if required. The solution is diluted further with sufficient quantity of water to make up volume of 1ml containing 75 mg of Diclofenac Sodium.

This solution is then filtered by using 0.2 micron membrane in aseptic condition or terminally sterilized. The solution is maintained oxygen free by flushing through constant nitrogen bubbling and is stable under storage condition. This resultant solution is filled into multidose vials and ampoules for I.M or I.V use. This prepared solution consists about 29 % of nonaqueous solution and 71 % of aqueous solution.

### Example 5

Previously heated water maintained at 80-85°C is cooled to at room temperature in the suitable vessel. 50 % of water is then mixed with 25 % of Transcutol without addition of Benzyl alcohol .The solution is stirred while simultaneously by adding 7.5 % Diclofenac Sodium. The ingredients are mixed well and nitrogen is continuously bubbled to remove oxygen and then solution is diluted upto final volume with addition of required amount of water in sufficient quantity. This solution is filtered aseptically. The pH of the solution is maintained by any suitable alkalizer or optionally using suitable buffering agent.

This solution can be filled in ampoule & multidose vials under nitrogen blanket or can be terminal sterilized to make free from endotoxins, for injectable purpose for administering through I.M or I.V administration. This prepared solution consists about 25% of nonaqueous solution and 75 % of aqueous solution.

### Example 6

Water previously heated at above 80 to 85 °C is allowed to cool and about 5.0 % of water is added in a vessel with a presumed batch size, 4 % of Benzyl alcohol and 20 % of Transcutol is added into water. The solution was stirred with the addition of Diclofenac Sodium at a concentration of 7.5% .The ingredients are mixed well to get clear liquid dissolved. The solution is diluted further with sufficient quantity of water upto required final volume. This solution is aseptically filtered by using 0.22 micron membrane. pH of the solution is maintained into the range of 7.5 to 9 by suitable buffering agents like Tris buffer, sodium phosphate, potassium phosphate or other alkalizer like sodium hydroxide.

The viscosity of the solution is also found about 2.004 cps at 30°C, desirable for injectables. The final resultant solution can be filled in an ampoule & a multi dose vials under constant nitrogen blanket This solution can be used as injectable through I.M or I.V administration. This prepared solution consists about 24 % of nonaqueous solution and 76 % of aqueous solution.

### Example 7

Previously heated water is maintained at 80 to 85°C and cooled. 2 % of Benzyl alcohol and 20 % of Trariscutol is added into 50% of water in a suitable vessel. Solution is stirred constantly with the addition of 7.5% Diclofenac Sodium. The ingredients are mixed well to get dissolved. The solution is diluted further with sufficient quantity of water to make up final volume required to obtain 75mg Diclofenac Sodium in 1ml. This solution is filtered aseptically by using 0.22 micron membrane. The pH of the solution is found to be maintained optionally by suitable buffering agent or alkalizer.

The solution is maintained and is stable under storage condition. The viscosity of the solution is also found about 2.047 mPa·s (cps) at 30°C, desirable for injectables. This prepared solution consists about 22% of nonaqueous solution and 78 % of aqueous solution. The resultant solution is poured into the ampoules of different volumes as per physician need & multi dose vials under nitrogen flush for injectable use as I.M or I.V injection.

### Example 8

Water is heated in a suitable vessel and cooled to room temperature. 50% of water is taken in a vessel and mixed with 20% Transcutol. To this mixture, 7.5% of Diclofenac Sodium is added with constant stirring followed by addition of suitable buffering agents like Tris buffer or phosphate buffers like monosodium phosphate, disodium phosphate or potassium phosphate or alkalizer like NaOH to adjust the pH of solution till the required range of 7.5 - 9. The solution is further diluted with sufficient quantity of water up to required concentration of 75 mg in 1 ml. The process is carried under constant nitrogen flushing. The final composition is filtered aseptically using 0.22 micron filter. The final resultant liquid is filled in an ampoule or multiple dose vials that can be used as injectables through I.M or I.V route of administration.

This prepared solution consists about 20% of nonaqueous solution and 80 % of aqueous solution.

### Example 9

In a suitable vessel water is heated at 80- 85°C and brought to room temperature. About 50% of composition, 15 % Transcutol and 4% of Benzyl alcohol is mixed into previously cooled water in the vessel. While stirring the solution, 7.5% Diclofenac Sodium is added and mixed properly till all the ingredients are mixed well and dissolved. Optionally pH is adjusted in required range by suitable buffering agent or alkali like sodium hydroxide. The solution is diluted further into water to the final volume in q.s. The same solution is filtered ascetically or terminally sterilized.

This prepared solution consists about 19% of nonaqueous solution and 81 % of aqueous solution. This solution is filled into in an ampoule or multidose vials as an injectable solution for administration through I.M and I.V route.

### Example 10

Diclofenac Sodium at a concentration of 7.5 % is added to 15 % Transcutol while stirring. 2 % Benzyl alcohol is added while stirring. The ingredients are mixed well to dissolve. While water is heated and brought to room temperature in a suitable vessel. This water upto 50 % is added into the above mixture and stirred continuously to get clear solution. The pH is adjusted with Tris buffer, phosphate buffers or alkalizer like sodium hydroxide and other. The solution is diluted further
q.s. with water to the final volume: This prepared solution consists about 17 % of nonaqueous solution and 83 % of aqueous solution.

This prepared solution is filtered aseptically or terminally sterilized and can be filled into ampoule & vials under the nitrogen flush and can be used as injectables for I.M or I.V route of administration. The final prepared liquid is poured into different size ampoules & multidose vials under nitrogen. This prepared solution consists about 17 % of nonaqueous solution and 83 % of aqueous solution.

### Example 11

Water is heated in a suitable vessel at 80 - 85 °C and cooled at room temperature. In 50 % of cool water, add 10 % Transcutol and 10 % Benzyl alcohol while stirring it simultaneously. Diclofenac Sodium about 7.5 % is added into this solution mixture. The ingredients are mixed well till all the ingredients get dissolved. The solution is diluted further with sufficient quantity of water to the volume. The desired pH upto 7.5 - 9 is obtained optionally by suitable buffer like Tris buffer, sodium or potassium phosphate buffers or alkalizer sodium hydroxide .The same is filtered aseptically or terminally sterilized and can be into filled in suitable ampoule & multidose vials by passing under nitrogen gas.

This prepared solution consists about 20 % of nonaqueous solution and 80 % of aqueous solution. The solution prepared by this process can be used for injection for administration through I.M or I.V route.

### Example 12

In a vessel, the solution is prepared comprising of Diclofenac Sodium at a concentration of 7.5 % by addition of 10 % Transcutol with 2 % Benzyl alcohol into 50 % of previously heated and cooled water and stirred well. The pH is adjusted in desired range if required, by Tris buffer or any other suitable buffer like sodium or potassium phosphate buffer or sodium acetate buffer. The solution is diluted further with water to the make up volume. The final volume solution is filtered aseptically or terminally sterilized. The filtration is carried by using 0.22 micron filter membrane.

### Example 13

Water is heated in a suitable vessel and cooled to room temperature. 50% of water is taken in a vessel and mixed with 5% Transcutol and 4% Benzyl alcohol. To this mixture, 7.5% of Diclofenac Sodium is added with constant stirring followed by addition of suitable buffering agents like Tris buffer/ phosphate buffers like sodium phosphate or alkalizer like NaOH to adjust the pH of solution till the required range of 7.5 - 9. The solution is further diluted with sufficient quantity of water up to required concentration of 75 mg in 1 ml. The process is carried under constant nitrogen flushing. The final composition is filtered aseptically using 0.22 micron filter. The final resultant liquid is filled in an ampoule or multiple dose vials.

This prepared solution consists about 9 % of non-aqueous solution and 91 % of aqueous solution.

Similarly, formulations no.14 & 15 were prepared by same procedure as given in above examples. For formulation 15, the viscosity is measured to be 3.40 mPa·s (cps) at 30°C.

### Example 14

Similarly further formulations were prepared for solution of Diclofenac Sodium after optimization of Transcutol and Benzyl alcohol range for stable clear solution with inclusion of additional excipients like antioxidant Sodium metabisulphite in variable concentration in addition to presence of Benzyl alcohol, alkalizer or buffers like Tris buffer or phosphate buffers like mono or di - sodium phosphate or potassium phosphate buffer and chelating agent.

Diclofenac formulations were prepared with inclusion of additional excipients like antioxidant Sodium metabisulphite in variable concentration in addition to presence of Benzyl alcohol, alkalizer or buffers and chelating agent. All the formulations were clear and stable (Table 3).

**Table 3: Formulations using Sodium metabisulphite and EDTA in 3.75% and 7.5% Diclofenac Sodium preparations:**

| **Formulation No.** | **Diclofenac Sodium concentration (%)** | **Transcutol concentration (%)** | **Benzyl alcohol concentration (%)** | **EDTA concentration (%)** | **Sodium metabisulphite (%)** | **Water for injection** |
|---|---|---|---|---|---|---|
| 33 | 3.75 | 10 | 2 | 0 | 0.2 | q.s. |
| 34 | 7.5 | 20 | 2 | 0 | 0.01 | q.s |
| 35 | 7.5 | 20 | 2 | 0 | 0.2 | q.s |
| 36 | 7.5 | 20 | 2 | 0.05 | 0.2 | q.s |
| 37 | 7.5 | 20 | 2 | 0.01 | 0.3 | q.s |
| 38 | 7.5 | 20 | 2 | 0.05 | 0.3 | q.s |
| 39 | 7.5 | 20 | 2 | 0.05 | 0.5 | q.s |
| 40 | 7.5 | 20 | 2 | 0.05 | 0.75 | q.s |
| 41 | 7.5 | 20 | 2 | 0.05 | 1.0 | q.s |
| 42 | 7.5 | 20 | 15 | 0 | 0.2 | q.s |
| 43 | 7.5 | 50 | 15 | 0 | 0.2 | q.s |

In the above formulations 33 to 43, Sodium metabisulphite is added as antioxidant in variable concentration from 0.01 - 1 %. Formulation 33 has a viscosity of about 1.25 mPa·s (cps) at 30°C, desirable for injectables.

For preparing the formulations given in table 3 above, water is heated in a suitable vessel to 80 to 85 °C . After cooling it to room temperature, 40 % of water is mixed with Transcutol in range of 5 to 30 %, most appropriately in 20 % concentration and 2 % Benzyl alcohol. 3.75% or 7.5 % of Diclofenac Sodium is then added into this solution with proper stirring. After dissolving Diclofenac Sodium, this solution is admixture with variable concentration of Sodium metabisulphite in 0.01, 0.2, 0.3, 0.5, 0.75 and 1 % respectively. EDTA about 0 % (formulations 33-35),0.01 % (formulation 37) to 0.05 % (formulations 36,38 to 41) is added optionally as chelating agent to prevent metallic ion impurity into the solution and to make the solution stable for longer storage. Then it is further diluted with remaining amount of water in sufficient amount to get a stable solution. If required, buffer like phosphate buffers (sodium phosphate) or alkalizer like 1M sodium hydroxide solution can be also added to adjust the required pH ranging between 7.5 to 9, preferably range between 8 to 8.6. This solution is filtered aseptically and sterilized to prevent endotoxins growth and removing oxygen under the constant flush of nitrogen gas so that the prepared solution can be made available for filling into ampoules and multidose vials. Solution prepared is clear and stable. The resultant liquid solution consist 37.5 mg/ml or 75 mg/ml of Diclofenac Sodium may be filed in 1- 3ml of ampoules and may be used as injection solution for administration through I.M or I.V route.

The above batches were found to be stable when kept for stability study according ICH guidelines. Test for assay and relative impurity test were performed. The satisfactory results were obtained for the prepared batches and final composition is selected on the basis of best result obtained from the above examples.

### Example 15

Further trials were carried out to prepare formulations comprising Diclofenac in combination with other anti-inflammatory, analgesic, and/ or anti-pyretic agents. Tables (4-5) below mention aqueous formulations of Paracetamol (formulation 44) and Dicyclomine HCl (formulation 45) in combinations of Diclofenac.

Similarly, different compositions with different Diclofenac concentration, its different salts or its free acid and number of antioxidants may be added. The trials can be carried out by a skilled chemist for preparation of the above aqueous solution for Diclofenac Sodium as well as in combination with alike NSAIDs like Paracetamol (formulation 44) and Dicyclomine HCl (formulation 45). Antioxidants like methylparaben, propylparaben, thiomersal, thioglycerol, ascorbyl palmitate, mixed tocopheryl ingredient, BHT, BHA, in addition to benzyl alcohol may also be used in preparation of Diclofenac salt solution in Transcutol in all possible dosage forms. In this preparation, a skilled chemist can use Transcutol in range of 5 % to 50 % and benzyl alcohol in range of 0 % to 10 % to prepare the solution with above preservatives in their required concentration.

**Table 4: Composition of Paracetamol 75 mg + Diclofenac Sodium 25 mg per ml Injection:**

| **Sr.no** | **Ingredients** | **Qty/ml** |
|---|---|---|
| 1 | Paracetamol | 75mg |
| 2 | Diclofenac Sodium | 25mg |
| 3 | Benzyl alcohol | 2% |
| 4 | Transcutol | 40% |
| 5 | EDTA | 0.02% |
| 6 | Sodium metabisulphite | 0.2% |
| 7 | Sodium hydroxide 1M | (for pH) |
| 8 | Water for injection | q.s |

In a suitable vessel, dissolve Sodium EDTA & Sodium metabisulphate in WFI and dissolve with stirring in another flask, add Diclofenac Sodium at a concentration of 2.5% & Paracetamol at a concentration of 7.5% and add Transcutol 40%, stirred well till the API get well dissolved than mix both solution and add benzyl alcohol. The pH of solution is adjusted in required range if required, by suitable buffering agent like 1M Sodium hydroxide and optionally other alkalizer like tris buffer or phosphate buffer which can be used. pH of the same was adjusted alkaline to 8.7(acceptable range 7.5 to 9). The solution is diluted further till final volume by sufficient quantity of WFI. The final volume solution is sterilized aseptically and poured into different fill volumes ampoules & multi dose vials under continuous nitrogen blanket. This prepared solution consists about 40 % of nonaqueous solution and 60 % of aqueous solution. The viscosity of the solution is about 3.7 mPa·s (cps) at 30°C.

The solubility of Dicyclomine Hydrochloride was found to be about 100 mg in 5 ml of Transcutol, 100 mg in 0.3 ml benzyl alcohol and 100 mg in 2.5 ml water for injection. This solubility profile can be beneficial to formulate this API in combination with Diclofenac Sodium as Diclofenac Sodium 50 mg + Dicyclomine Hydrochloride 20 mg filled as 0.5ml, 1ml, 2 ml solution in the suitable ampoules.

**Table 5: Composition of Diclofenac Sodium 50 mg + Dicyclomine HCl 20 mg per ml Injection:**

| **Sr.no** | **Ingredients** | **Qty/ml** |
|---|---|---|
| 1 | Diclofenac Sodium | 50mg (5%) |
| 2 | Dicyclomine HCl | 20mg (2%) |
| 3 | Transcutol | 50% |
| 4 | Water for injection | q.s |

In a suitable vessel, the solution is prepared comprising of Diclofenac Sodium at a concentration of 5% by addition of 50 % Transcutol and dissolve Dicyclomine Hydrochloride 2% in water for injection and stirred well till the API get well dissolved add sodium metabisulphite and mix Diclofenac sodium & Dicyclomine solution, add benzyl alcohol. The pH of solution is adjusted in the required range near to 7.5 i.e neutralized to 6.84 by adding suitable buffering agent like sodium hydroxide and optionally other alkalizer like tris buffer or phosphate buffers. The solution is diluted further till final volume by sufficient quantity of WFI. The final volume solution is sterilized aseptically and poured into ampoules of 1 ml volume capacity or 0.5ml or 2ml capacity as per need of physicians under continuous nitrogen blanket. The viscosity of the solution is also found about 4.74 mPa·s (cps) at 30°C, desirable for injectables.

This prepared solution consists about 50 % of nonaqueous solution and 50 % of aqueous solution. This formulation can be useful for the treatment of acute colicky and pain caused as intestinal, biliary and ureteric, spasmodic dysmenorrheal in the adults.

### Example 16: Pharmacokinetic study

The pharmacokinetic study of one of the product prepared according to the present invention (herein referred as "inventive/test product") was carried out against another marketed diclofenac composition DYNAPAR AQ (Troika Indian patent 231479; US equivalent 20080153914) that was used as a reference. DYNAPAR AQ which is used as reference product comprises Diclofenac (75 mg), Glycofurol and benzyl alcohol. The sample prepared according to the present invention (test product) has the following composition: Diclofenac sodium: 7.5 % (i.e., 75 mg), Transcutol: 20 %, Benzyl alcohol: 2 %, pH: 7.5 to 9.0. The bioequivalence studies were conducted at Clinical Research Centre.

The mean Cₘₐₓ and Tₘₐₓ, for the test and reference formulations were 2219.1498 ± 962.7785 ng/ml, 1790.4493 ± 973.7963 ng/ml and 0.6250 ± 0.2516 h, 0.7350 ± 0.3283 h respectively. AUC values from 0 to last measurable time point 't' observed for test and reference formulations were 4620.6682 ± 1106.9752 ng.h/ml and 3896.7670 ± 988.0809 ng.h/ml respectively. The mean AUC 0 to infinity for test and reference formulations was 4800.2394 ± 1106.9752 ng.h/ml and 4165.6212 ± 1128.9737 ng.h/ml.

Results suggest that Cₘₐₓ: the maximum concentration available in the blood for inventive product is higher than the marketed product and Tₘₐₓ: the time to reach maximum concentration in the blood for the present inventive drug is short than that of the marketed product. While AUC both 0 to infinity and 0 to time point t for test product shows the maximum concentration of the drug is effectively available in plasma and it also indicates better extent and rate of absorption of drug than that of marketed product. The Cₘₐₓ observed in the prepared injectable was found to be safe and doesn't exceed toxicity level. It is found to be better than that of currently available product. The time for reaching maximum plasma concentration of drug (Tmax) by the prepared injectable was less than the currently available product which indicates that the present test product is effective at short period of time. Hence, onset of action of the present invention product is more rapid than the marketed product. This indicates that the present investigation product shows rapid penetration & better absorption as compare to the marketed product when given by Intramuscular route.

Thus, this indicates that use of Transcutol in said range in the injectable preparation of Diclofenac Sodium provides better pharmacokinetic profile and effective pharmacologic activity with rapid onset of action in comparison to the currently available brand. The bioavailability of drug is higher by the present invention injection. This shows advantageous use of Transcutol.

The following data (tables 6-8) given below provides information of parameters of Pharmacokinetic study through the mean plasma concentration vs. time curve for reference marketed (R) and test (T) product:

**Table 6: Pharmacokinetic Parameters of Diclofenac Sodium Injection 75 mg/1ml**

| **(Reference/ Marketed product)** | | | | | | |
|---|---|---|---|---|---|---|
| **Parameters** | **t_{1/2} (hr)** | **kₑₗ (1/hr)** | **tₘₐₓ (hr)** | **Cₘₐₓ (ng/ml)** | **AUC₀₋ₜ (ng.h/ml)** | **AUC_{0-α} (ng.h/ml)** |
| N | 6 | 6 | 6 | 6 | 6 | 6 |
| Mean | 2.5092 | 0.3487 | 0.7350 | 1790.4493 | 3896.7670 | 4165.6212 |
| SD | 1.5177 | 0.1573 | 0.3283 | 973.7963 | 988.0809 | 1128.9737 |
| CV% | 60.48 | 45.11 | 44.67 | 54.39 | 25.36 | 27.10 |
| Geometric Mean | 2.207 | 0.314 | 0.659 | 1612.043 | 3777.574 | 4018.889 |

**Table 7: Pharmacokinetic Parameters of Diclofenac Sodium Injection 75 mg/1 ml**

| **(Inventive product)** | | | | | | |
|---|---|---|---|---|---|---|
| **Parameters** | **t_{1/2} (hr)** | **kₑₗ (1/hr)** | **tₘₐₓ (hr)** | **Cₘₐₓ (ng/ml)** | **AUC₀₋ₜ (ng.h/ml)** | **AUC_{0-α} (ng.h/ml)** |
| N | 6 | 6 | 6 | 6 | 6 | 6 |
| Mean | 2.0928 | 0.3564 | 0.6250 | 2219.1498 | 4620.6682 | 4800.6682 |
| SD | 0.6652 | 0.1004 | 0.2516 | 962.7785 | 1106.9752 | 1128.7644 |
| CV% | 31.79 | 28.17 | 40.26 | 43.39 | 23.96 | 23.51 |
| Geometric Mean | 2.015 | 0.344 | 0.582 | 2039.106 | 4510.446 | 4688.917 |

**Table 8: Comparison of Mean value of Parameters of Diclofenac Sodium Injection 75 mg/1 ml**

| **(Inventive product vs. Reference product)** | | |
|---|---|---|
| **Parameters** | **Diclofenac Sodium Injection 75 mg/1 ml** | |
| | **Mean ± SD** | |
| | **Inventive product** | **Reference/Marketed product** |
| Cₘₐₓ (ng/ml) | 2219.1498 ± 962.7785 | 1790.4493 ± 973.7963 |
| AUC₀₋ₜ (ng.h/ml) | 4620.6682 ± 1106.9752 | 3896.7670 ± 988.0809 |
| AUC_{0-α} (ng.h/ml) | 4800.2394 ± 1106.9752 | 4165.6212 ± 1128.9737 |
| tₘₐₓ (hr) | 0.6250 ± 0.2516 | 0.7350 ± 0.3283 |
| kₑₗ (1/hr) | 0.3564 ± 0.1004 | 0.3487 ± 0.15173 |
| t_{1/2} (hr) | 2.0928 ± 0.6652 | 2.5092 ± 1.5177 |

## Claims

1. An aqueous injectable composition comprising 25-200mg/mL of Diclofenac or salts thereof, 5-50% (wt/v) of diethylene glycol monoethyl ether and water as a principal solvent in at least 0.5ml of solution, wherein said composition has a viscosity between about 1 to about 5 mPa·s (cps) at 30°C.

2. The composition as claimed in claim 1, wherein Diclofenac salt is selected from Diclofenac sodium, Diclofenac potassium, Diclofenac diethylamine, Diclofenac diethanolamine or Diclofenac beta-dimethyl aminoethanol.

3. The composition as claimed in claim 1, wherein said composition comprises benzyl alcohol optionally in a concentration of not more than 3% (v/v).

4. The composition as claimed in claim 1, wherein the composition also comprises solvents/ co-solvents and preservatives,
optionally wherein the solvents/ co-solvents and preservatives are selected from the group consisting of alcohols, parabens, sodium metabisulphite, sodium bisulphite, sodium sulphite, propylene glycol, thiomerosal, glycerol and thioglycerol or a combination thereof, ascorbyl palmitate, ascorbate, tocopherol alpha, alpha-tocopherol hydrogen succinate and mixture of tocopheryl derivatives, thioglycolate sodium.

5. The composition as claimed in claim 1, wherein the preservative is sodium metabisulphite and is present in a concentration between 0.1 - 1 %.

6. The composition as claimed in claim 1, wherein said composition comprises chelating agents selected from a group consisting of sodium EDTA (ethylenediaminetetra acetic acid), disodium EDTA, Calcium disodium EDTA, Calcium versetamide Na, Calteridol, DTPA (Diethylenetriaminepenta acetic acid), optionally wherein the amount of chelating agent used is between 0.01-0.05%

7. The composition as claimed in any preceding claim, wherein the composition is prepared as unit dose bolus injection or multi dose vials for administration.

8. The composition as claimed in any preceding claim, wherein the composition is prepared in 1 - 5 ml, preferably 1-3 ml of solution and may be further diluted prior to use.

9. The composition as claimed in any preceding claim, wherein the composition comprises 25-200mg of Diclofenac or salts thereof, 5-50% (wt/v) of diethylene glycol monoethyl ether in combination with water as a principal solvent in 1ml of solution.

10. The composition as claimed in any preceding claim, wherein pH of said composition is between 7.5-9.0.

11. The composition as claimed in claim 1, wherein said composition further comprises therapeutically effective amount of other pharmaceutically active ingredients selected from anti-inflammatory, analgesic, and/ or anti-pyretic agents.

12. The composition as claimed in claim 1, wherein said composition further comprising antioxidants selected from the group consisting of thioglycerols, acetyl cysteine, butylated hydroxyanisole (BHA) and butylated hydroxytoluene (BHT), ascorbates, ascorbyl palmitate, methylparaben, Propylparaben, thiomersal and mixed Tocopheryl ingredient.

13. The composition as claimed in claim 1, wherein the composition further comprises therapeutically effective amount of dicyclomine hydrochloride.

14. The composition as claimed in claim 1, wherein the composition further comprises therapeutically effective amount of paracetamol.

15. A method of preparation of an injectable aqueous composition according to any one of claims 1-14, comprising Diclofenac or salts thereof and diethyleneglycol monoethyl ether, said method comprises:
a. preparing an aqueous solution by adding 30-50 % of water to 5-50% of diethylene glycol monoethyl ether;
b. adding 25 mg-200mg/mL of Diclofenac in said aqueous solution with constant stirring under nitrogen atmosphere;
c. adjusting pH of said solution in a range of 7.5-9.0; and
d. diluting said solution to achieve desired concentration of Diclofenac in said solution.

16. A composition for use in the treatment or prevention of pain and/or inflammation conditions like headache, sore throat, various musculoskeletal and joint disorders like rheumatoid arthritis, osteoarthritis, ankylosing spondylitis, peri-articular disorders like bursitis and tendinitis, soft tissue disorders like sprains and strains, painful condition such as renal colic, acute gout, dysmenorrhoea, migraine and some surgical procedures, in management of actinic keratosis and fever, post operative pain, for juvenile idiopathic arthritis, acute postoperative pain, intra-operative miosis, for treatment of inflammation after surgery or during surgical procedures, pain control of total hip replacement arthroplasty, the composition comprising Diclofenac or salts thereof from 25 mg-200mg/mL in 5-50% (wt/v) diethylene glycol monoethyl ether and water as a principal solvent in at least 0.5ml of solution, wherein said composition has a viscosity between about 1 to about 5 mPa·s (cps) at 30°C.

## Patentansprüche

1. Wässrige injizierbare Zusammensetzung, umfassend 25-200 mg/ml Diclofenac oder Salze davon, 5-50 % (Gew./Vol.) Diethylenglycolmonoethylether und Wasser als ein Hauptlösungsmittel in mindestens 0,5 ml Lösung, wobei die Zusammensetzung eine Viskosität zwischen etwa 1 bis etwa 5 mPa·s (cps) bei 30 °C aufweist.

2. Zusammensetzung nach Anspruch 1, wobei das Diclofenac-Salz ausgewählt ist aus Diclofenac-Natrium, Diclofenac-Kalium, Diclofenac-Diethylamin, Diclofenac-Diethanolamin oder Diclofenac-Beta-dimethylaminoethanol.

3. Zusammensetzung nach Anspruch 1, wobei die Zusammensetzung Benzylalkohol optional in einer Konzentration von nicht mehr als 3 % (Vol./Vol.) umfasst.

4. Zusammensetzung nach Anspruch 1, wobei die Zusammensetzung auch Lösungsmittel/Hilfslösungsmittel und Konservierungsmittel umfasst,
wobei optional die Lösungsmittel/Hilfslösungsmittel und Konservierungsmittel aus der Gruppe ausgewählt sind, die aus Alkoholen, Parabenen, Natriummetabisulfit, Natriumbisulfit, Natriumsulfit, Propylenglycol, Thiomersal, Glycerol und Thioglycerol oder einer Kombination davon, Ascorbylpalmitat, Ascorbat, Tocopherol alpha, Alpha- Tocopherol-Hydrogensuccinat und Mischung aus Tocopheryl-Derivaten, Thioglycolat-Natrium besteht.

5. Zusammensetzung nach Anspruch 1, wobei das Konservierungsmittel Natriummetabisulfit ist und in einer Konzentration zwischen 0,1 - 1 % vorliegt.

6. Zusammensetzung nach Anspruch 1, wobei die Zusammensetzung Chelatbildner umfasst, die aus einer Gruppe ausgewählt sind, die aus Natrium-EDTA (Ethylendiamintetraessigsäure), Dinatrium-EDTA, Calcium-Dinatrium-EDTA, Calcium-Versetamid-Na, Calteridol, DTPA (Diethylentriaminpentaessigsäure) besteht, wobei optional die Menge des verwendeten Chelatbildners zwischen 0,01-0,05 % beträgt.

7. Zusammensetzung nach einem der vorstehenden Ansprüche, wobei die Zusammensetzung als Einzeldosis-Bolusinjektion oder Mehrfachdosis-Fläschchen zur Verabreichung hergestellt ist.

8. Zusammensetzung nach einem der vorstehenden Ansprüche, wobei die Zusammensetzung in 1 - 5 ml, vorzugsweise 1 -3 ml Lösung hergestellt ist und vor der Verwendung weiter verdünnt werden kann.

9. Zusammensetzung nach einem der vorstehenden Ansprüche, wobei die Zusammensetzung 25-200 mg Diclofenac oder Salze davon, 5-50 % (Gew./Vol.) Diethylenglycolmonoethylether in Kombination mit Wasser als Hauptlösungsmittel in 1 ml Lösung umfasst.

10. Zusammensetzung nach einem der vorstehenden Ansprüche, wobei der pH-Wert der Zusammensetzung zwischen 7,5-9,0 liegt.

11. Zusammensetzung nach Anspruch 1, wobei die Zusammensetzung ferner eine therapeutisch wirksame Menge anderer pharmazeutisch wirksamer Inhaltsstoffe umfasst, die aus entzündungshemmenden, analgetischen und/oder antipyretischen Mitteln ausgewählt sind.

12. Zusammensetzung nach Anspruch 1, wobei die Zusammensetzung ferner Antioxidantien umfasst, die aus der Gruppe ausgewählt sind, die aus Thioglycerolen, Acetylcystein, Butylhydroxyanisol (BHA) und Butylhydroxytoluen (BHT), Ascorbaten, Ascorbylpalmitat, Methylparaben, Propylparaben, Thiomersal und gemischtem Tocopheryl-Inhaltsstoff besteht.

13. Zusammensetzung nach Anspruch 1, wobei die Zusammensetzung ferner eine therapeutisch wirksame Menge von Dicyclomin-Hydrochlorid umfasst.

14. Zusammensetzung nach Anspruch 1, wobei die Zusammensetzung ferner eine therapeutisch wirksame Menge von Paracetamol umfasst.

15. Verfahren zur Herstellung einer injizierbaren wässrigen Zusammensetzung nach einem der Ansprüche 1-14, umfassend
Diclofenac oder Salze davon und Diethylenglycolmonoethylether, wobei das Verfahren Folgendes umfasst:
a. Herstellen einer wässrigen Lösung durch Zugeben von 30-50 % Wasser zu 5-50 % Diethylenglycolmonoethylether;
b. Zugeben von 25 mg-200 mg/ml
Diclofenac zu der wässrigen Lösung bei konstantem Röhren unter Stickstoffatmosphäre;
c. Einstellen des pH-Werts der Lösung in einem Bereich von 7,5-9,0; und
d. Verdünnen der Lösung, um die erwünschte Konzentration von Diclofenac in der Lösung zu erhalten.

16. Zusammensetzung zur Verwendung bei der Behandlung oder Vorbeugung von Schmerz- und/oder Entzündungszuständen wie Kopfschmerzen, Halsschmerzen, verschiedenen Muskel-Skelett- und Gelenkstörungen wie rheumatoider Arthritis, Osteoarthritis, ankylosierender Spondylitis, periartikulären Störungen wie Bursitis und Tendinitis, Weichteilstörungen wie Verstauchungen und Zerrungen, schmerzhaften Zuständen wie Nierenkolik, akuter Gicht, Dysmenorrhoea, Migräne und einigen operativen Eingriffen, bei der Kontrolle von aktinischer Keratose und Fieber, postoperativen Schmerzen, für juvenile idiopathische Arthritis, akute postoperative Schmerzen, intraoperative Miosis, zur Behandlung von Entzündung nach Operation oder während operativer Verfahren, Schmerzkontrolle bei vollständiger Hüftersatzgelenkplastik, wobei die Zusammensetzung Diclofenac oder Salze davon von 25 mg-200 mg/ml in 5-50 % (Gew./Vol.) Diethylenglycolmonoethylether und Wasser als Hauptlösungsmittel in mindestens 0,5 ml Lösung umfasst, wobei die Zusammensetzung eine Viskosität zwischen etwa 1 bis etwa 5 mPa·s (cps) bei 30 °C aufweist.

## Revendications

1. Composition injectable aqueuse comprenant entre 25 et 200 mg/mL de Diclofénac ou de sels de celui-ci, entre 5 et 50 % (en poids/volume) de l'éther monoéthylique du diéthylène glycol et d'eau en tant que solvant principal dans au moins 0,5 ml de solution, ladite composition comportant une viscosité comprise entre environ 1 et environ 5 mPas (cps) à 30 °C.

2. Composition selon la revendication 1, dans laquelle un sel de Diclofénac est choisi parmi le Diclofénac sodique, le Diclofénac potassique, le Diclofénac diéthylamine, le Diclofénac diéthanolamine ou le Diclofénac béta-diméthylaminoethanol.

3. Composition selon la revendication 1, dans laquelle ladite composition comprend un alcool benzylique en concentration éventuellement inférieure ou égale à 3 % (en volume).

4. Composition selon la revendication 1, dans laquelle la composition comprend également des solvants/co-solvants et des agents de préservation, dans laquelle les solvants/co-solvants et agents de préservation sont éventuellement choisis dans le groupe constitué des alcools, des parabens, du métabisulphite de sodium, du bisulfite de sodium, du sulfite de sodium, du propylène glycol, du thiomérosal, du glycérol et du thioglycérol ou d'une combinaison de ceux-ci, du palmitate d'ascorbyle, de l'ascorbate, du tocophérol alpha, du succinate d'hydrogène de l'alpha-tocophérol et d'un mélange de dérivés de tocophéryle, du thioglycolate de sodium.

5. Composition selon la revendication 1, dans laquelle l'agent de préservation est le métabisulfite de sodium et est présent en une concentration comprise entre 0,1 et 1 %.

6. Composition selon la revendication 1, dans laquelle ladite composition comprend des agents de chélation choisis dans un groupe constitué de l'EDTA sodé (acide éthylènediaminetétraacétique), de l'EDTA disodé, de l'EDTA calcique disodé, du versétamide calcique de Na, du Caltéridol, du DTPA (acide diéthylènetriaminepentaacétique), dans lequel la quantité d'agent de chélation éventuellement utilisée est comprise entre 0,01 et 0,05 %.

7. Composition selon l'une quelconque des revendications précédentes, dans laquelle la composition est préparée comme une injection de bolus en doses individuelles ou en flacons multidoses destinée à son administration.

8. Composition selon l'une quelconque des revendications précédentes, dans laquelle la composition est préparée dans 1 à 5 ml, de préférence dans 1 à 3 ml de solution et peut en outre être diluée avant son utilisation.

9. Composition selon l'une quelconque des revendications précédentes, dans laquelle la composition comprend entre 25 et 200 mg de Diclofénac ou de sels de celui-ci, entre 5 et 50 % (en poids/volume) d'éther monoéthylique du diéthylène glycol en combinaison avec de l'eau en tant que solvant principal dans 1 ml de solution.

10. Composition selon l'une quelconque des revendications précédentes, dans laquelle le pH de ladite composition est compris entre 7,5 et 9,0.

11. Composition selon la revendication 1, dans laquelle ladite composition comprend en outre une quantité thérapeutiquement efficace d'autres ingrédients pharmaceutiquement actifs choisis parmi des agents anti-inflammatoires, analgésiques et/ou antipyrétiques.

12. Composition la revendication 1, ladite composition comprenant en outre des antioxydants choisis dans le groupe constitué de thioglycérols, de l'acétyl cystéine, de l'hydroxyanisole butylée (BHA) et de l'hydroxytoluène butylé (BHT), des ascorbates, du palmitate d'ascorbyle, du méthylparaben, du propylparaben, du thiomersal et d'ingrédient du Tocophéryle mélangé.

13. Composition de stérilisation selon la revendication 1, dans laquelle la composition comprend en outre une quantité thérapeutiquement efficace de chlorhydrate de dicyclomine.

14. Composition de stérilisation selon la revendication 1, dans laquelle la composition comprend en outre une quantité thérapeutiquement efficace de paracétamol.

15. Procédé de préparation d'une composition aqueuse injectable selon l'une quelconque des revendications 1 à 14, comprenant
du Diclofénac ou des sels de celui-ci et de l'éther monoéthylique du diéthylène glycol, ledit procédé comprend :
a. la préparation d'une solution aqueuse par l'ajout de 30 à 50 % d'eau à 5-50 % d'éther monoéthylique du diéthylène glycol,
b. l'ajout de 25 mg - 200 mg/mL de Diclofénac dans ladite solution aqueuse avec une agitation continue sous une atmosphère d'azote ;
c. le réglage du pH de ladite solution dans une plage comprise entre 7,5 et 9,0 ; et
d. la dilution de ladite solution pour obtenir la concentration souhaitée de Diclofénac dans ladite solution.

16. Composition destinée à être utilisée dans le traitement ou la prévention de la douleur et/ou des affections inflammatoires tels que les maux de tête, les maux de gorge, de nombreux troubles musculosquelettiques ou articulatoires tels que l'arthrite rhumatoïde, l'ostéoarthrite, la spondylite ankylosante, des troubles périarticulaires tels que la bursite et la tendinite, des troubles des tissus tel que des entorses et des foulures, une affection douloureuse telle que la colique rénale, la goutte aiguë, la dysménorrhée, la migraine et certaines procédures chirurgicales, dans la gestion de la kératose actinique et de la fièvre, des douleurs postopératoires, pour l'arthrite idiopathique juvénile, des douleurs postopératoires aiguës, la myosis intra-opératoire, pour le traitement de l'inflammation après chirurgie ou pendant des procédures chirurgicales, le contrôle de la douleur de l'arthroplastie du remplacement total de la hanche, la composition comprenant du Diclofénac ou des sels de celui-ci de 25 mg - 200 mg/mL dans 5-50 % (en poids/volume) d'éther monoéthylique du diéthylène glycol et d'eau en tant que solvant principal dans au moins, 0.5 ml de solution, ladite solution comprenant une viscosité comprise entre environ 1 et environ 5 mPas (cps) à 30 °C.
